## Europäisches Patentamt

## European Patent Office

## Office européen des brevets

(19)

(11) Veröffentlichungsnummer: **0 216 873**
**B1**

(12) # EUROPÄISCHE PATENTSCHRIFT

(45) Veröffentlichungstag der Patentschrift:
**13.09.89**

(21) Anmeldenummer: **86902270.7**

(22) Anmeldetag: **09.04.86**

(86) Internationale Anmeldenummer:
**PCT/AT 86/00030**

(87) Internationale Veröffentlichungsnummer:
**WO 86/06093 (23.10.86 Gazette 86/23)**

(51) Int. Cl.⁴: **C 12 M 1/00, C 12 P 7/06,
B 01 F 7/10, B 01 F 9/06**

(54) **EINRICHTUNG ZUR KONTINUIERLICHEN GEWINNUNG VON ÄTHANOL AUS VERGÄRBAREN ZUCKERLÖSUNGEN.**

(30) Priorität: **09.04.85 AT 1068/85**

(43) Veröffentlichungstag der Anmeldung:
**08.04.87 Patentblatt 87/15**

(45) Bekanntmachung des Hinweises auf die Patenterteilung:
**13.09.89 Patentblatt 89/37**

(84) Benannte Vertragsstaaten:
**AT BE CH DE FR GB IT LI LU NL SE**

(56) Entgegenhaltungen:
**EP-A-0 039 518
CH-A-376 089
CH-A-443 226
FR-A-1 563 514
US-A-4 359 533**

(73) Patentinhaber: **VOEST- ALPINE Aktiengesellschaft,
Muldenstrasse 5, A-4020 Linz (AT)**

(72) Erfinder: **CVITAS, Vilim, Wöberweg 8, A-4060 Linz
(AT)**
Erfinder: **FALTEJSEK, Karl, Lüfteneggerstrasse 6,
A-4020 Linz (AT)**
Erfinder: **HANKE, Reinhart, Annaberggasse 2,
A-8700 Leoben (AT)**
Erfinder: **KLINAR, Gottfried, Alpenstrasse 33,
A-8707 Leoben (AT)**

(74) Vertreter: **Haffner, Thomas M., Dr.,
Patentanwaltskanzlei Dipl.- Ing. Adolf
Kretschmer Dr. Thomas M. Haffner
Schottengasse 3a, A-1014 Wien (AT)**

## Beschreibung

Die Erfindung bezieht sich auf eine Einrichtung zur kontinuierlichen Gewinnung von Äthanol aus vergärbaren Zuckerlösungen mit einer Einrichtung zum Mischen von Zuckerlösung mit Hefe und zum Abtrennen von mit Zuckerlösung beladener Hefe und einem unter Unterdruck setzbaren Gärbehälter.

Aus der EP-A-44 428 ist es bereits bekannt, zur Herstellung von Alkohol aus Stärke oder stärkehaltigen Rohstoffen eine Schlempe zu gewinnen, welche durch Zerkleinern, thermischen Aufschluß und Verzuckerung der stärkehaltigen Rohstoffe gebildet wird. Es sind eine Reihe von zuckerhaltigen Rohstoffen bekannt, aus welchen unmittelbar Zuckerlösungen auf extraktivem Wege hergestellt werden können, und es ist bekannt, derartige Zuckerlösungen bis zur Glucose abzubauen und eine auf diese Weise erhaltene Maische zu vergären. Der Gärprozeß verlangt hiebei je nach den eingesetzten Rohstoffen bzw. der eingesetzten Maische unterschiedliche Bedingungen und erfordert einen relativ langen Reaktionszeitraum für den Abschluß der Gärung. Nachteilig bei den bekannten Gärverfahren ist, daß die Maischen bzw. gärfähigen Substrate im Verlauf der Gärung unterschiedliche Konzentrationen sowohl an vergärbarem Zucker als auch an Gärungsprodukten aufweisen, welche mit zunehmender Gärung die Reaktionsgeschwindigkeit bzw. die Gärungsgeschwindigkeit ungünstig beeinflussen.

Die Erfindung zielt nun darauf ab, eine Einrichtung der eingangs genannten Art zu schaffen, bei welcher in einfacher Weise unter Anwendung von unteratmosphärischem Druck gearbeitet werden kann, wobei Alkohol ohne übermäßige Schaumbildung destillativ abgetrennt werden kann, und zielt weiters darauf ab, das durchzuführende Gärverfahren in Stufen unterteilen zu können, um den jeweiligen Konzentrationen in Anpassung an die Verhältnisse der Maische und den kontinuierlichen Betrieb in optimaler Weise Rechnung tragen zu können. Zur Lösung dieser Aufgabe besteht die erfindungsgemäße Einrichtung im wesentlichen darin, daß der Gärbehälter aus mehreren über Druckschleusen, insbesondere Ventile oder Pumpen, verbindbaren rohrförmigen Behältern gebildet ist, daß die einzelnen rohrförmigen Behälter um die Achse derselben drehbare Scheiben aufweisen, welche zumindest teilweise in das Gärsubstrat eintauchen und mit der Achse Winkel von größer oder kleiner 90° einschließen, und daß die rohrförmigen Behälter gesonderte Gasableitungen aufweisen. Dadurch, daß der Gärbehälter in mehrere einzelne rohrförmige Behälter unterteilt wird, läßt sich in den verschiedenen rohrförmigen Behältern eine unterschiedliche Atmosphäre und vor allen Dingen unterschiedlicher Druck anwenden. Mit Rücksicht auf die bevorzugte Verfahrensweise, während der Gärung mit unteratmosphärischem Druck zu arbeiten, muß mit erhöhter Schaumbildung gerechnet werden. Ein derartiger während des Gärens gebildeter Schaum läßt sich in einfacher Weise dadurch zerstören bzw. seine Entstehung verhindern, daß die einzelnen rohrförmigen Behälter um die Achse derselben drehbare Scheiben aufweisen, welche die flüssige Phase in einer auf- und ab- bzw. hin- und hergehenden Bewegung halten. Zu diesem Zweck sind die Scheiben relativ zur Drehachse so orientiert, daß sie mit der Achse Winkel von größer oder kleiner 90° einschließen, wodurch ein pulsierender hydrostatischer Druck bewirkt wird, der die Ausgasung ohne Schaumbildung bewirkt. Dadurch, daß die einzelnen rohrförmigen Behälter über Druckschleusen miteinander verbunden sind, läßt sich in den einzelnen rohrförmigen Behältern unterschiedlicher Druck anwenden und es läßt sich eine bevorzugte Verfahrensführung verwirklichen, bei welcher der Druck stufenweise im Zuge des Verfahrens abgesenkt wird.

Ein analoger rohrförmiger Behälter mit rotierbar gelagerten Scheiben läßt sich im Rahmen der erfindungsgemäßen Einrichtung als Misch- und Trennvorrichtung vorschalten, wofür erfindungsgemäß vorgeschlagen wird, daß den Behältern eine gleichfalls einen rohrförmigen Behälter aufweisende Misch- und Trennvorrichtung vorgeschaltet ist, in welchem um die Behälterachse, gemeinsam mit dem Behälter oder gesondert, drehbare Scheiben angeordnet sind, welche im wesentlichen dichtend an den Innenumfang des rohrförmigen Behälters anschließen, daß die Scheiben in wenigstens einem Axialschnitt abwechselnd entgegengesetzt zur Achse geneigt angeordnet sind und jeweils eine dem Innenumfang des Behälters nahe Durchströmöffnung und wenigstens eine der Achse gegenüberliegende achsnahe Durchströmöffnung aufweisen, wobei in Achsrichtung gesehen achsnahe bzw. dem Innenumfang nahe Durchströmöffnungen an benachbarten Scheiben um 180° in Drehrichtung versetzt angeordnet sind, und daß der Behälter an einem Ende eine Aufgabeöffnung für Hefe und gegebenenfalls für Zuckerlösung und eine Austragsöffnung für abgereicherte Zuckerlösung oder beladenen Hefeschlamm und am anderen Ende gegebenenfalls eine Aufgabeöffnung für Zuckerlösung und eine Austragsöffnung für beladenen Hefeschlamm oder für abgereicherte Zuckerlösung aufweist. Die Trennung der aufschwimmenden Phase von der verbleibenden mit Zucker abgereicherten Phase erfolgt hiebei dadurch, daß die jeweils aufschwimmende Phase über achsnahe Durchbrechungen der Scheiben immer dann in eine benachbarte Kammer überströmt, wenn diese benachbarte Kammer auf Grund der Geometrie der Stellung der Scheiben einen niedrigeren Flüssigkeitsspiegel aufweist. Der Transport des Mediums erfolgt auf Grund des im wesentlichen dichtenden Anschlusses der Scheiben an die Innenwand des rohrförmigen Behälters immer von einer Kammer mit einem höheren Flüssigkeitsspiegel in eine Kammer mit niedrigerem Flüssigkeitsspiegel, wobei die Höhe des Flüssigkeitsspiegels vom Ausmaß der Annäherung bzw. der Entfernung benachbarter Scheiben im unteren Teilbereich des rohrförmigen Behälters abhängig ist. Umgekehrt erfolgt ein Transport der jeweiligen anderen Phase in die Gegenrichtung dadurch, daß jede Scheibe achsnahe und achsferne Durchströmöffnungen aufweist, welche an benachbarten Scheiben um 180° in Drehrichtung versetzt angeordnet sind. Es gelangt somit abwechselnd jeweils eine achsnahe bzw. eine achsferne Durchbrechung einer Scheibe bei einer Umdrehung derselben unter den Flüssigkeitsspiegel, wodurch der Überströmvorgang in die jeweils benachbarte Kammer mit entsprechend niedrigerem Flüssigkeitsspiegel erzielt wird. Da der Flüssigkeitsspiegel zyklisch an- und absteigt, ergibt sich auf Grund der Versetzung dieser Überströmöffnungen abwechselnd ein Transport der achsnahen Phase in eine Richtung und ein Transport der achsfernen Phase in die andere Richtung des rohrförmigen Behälters.

Gleichzeitig mit einer guten Durchmischung und Beladung der Hefe mit zu vergärendem Zucker kann somit mit einer derartigen Einrichtung in einfacher Weise eine Trennung der mit Zucker beladenen Hefe und der abgereicherten Zuckerlösung erzielt werden.

In vorteilhafter Weise ist die erfindungsgemäße Einrichtung so weitergebildet, daß die rohrförmigen Behälter des Gärbehälters die Eintrags- und Austragsöffnungen für beladene Hefe nahe den gegenüberliegenden Stirnseiten der Behälter aufweisen und daß die Scheiben Überströmöffnungen für den Transport des Gärsubstrates von der Eintragsöffnung zur Austragsöffnung aufweisen. Sofern die Bewegung der Scheiben unmittelbar zum Transport der flüssigen Phase von der Eintrags- zur Austragsöffnung herangezogen werden soll, ist ein weitgehend dichter Abschluß an den Innenmantel des Behälters erforderlich, um eine Niveaudifferenz zwischen benachbarten Kammern zu erzielen. Die Überströmöffnungen können aber hier ohne weiteres von den verbleibenden Ringspalten zwischen der Außenkontur der Scheiben und dem inneren Mantel der Behälter gebildet werden, sofern für die gerichtete Strömung externe Einrichtungen zur Verfügung gestellt werden. Wenn, wie es einer bevorzugten Weiterbildung der Erfindung entspricht, so gearbeitet wird, daß der Druck in aufeinanderfolgenden rohrförmigen Behältern stufenweise absenkbar ist, kann der Saugdruck des jeweils nachfolgenden Gärbehälters für den Abtransport des Mediums aus dem vorangehenden Gärbehälter herangezogen werden, wodurch sich bereits durch den unterschiedlichen Druck eine gerichtete Strömung erzielen läßt. In besonders vorteilhafter Weise wird in wenigstens drei Stufen gearbeitet und es werden drei rohrförmige Behälter hintereinandergeschaltet, wobei der Druck im ersten Behälter bevorzugt zwischen 0,75 und 0,95 bar absolut, im zweiten Behälter mit etwa 0,25 bis 0,50 bar absolut und im dritten Behälter mit 0,1 bis 0,25 bar absolut gewählt wird.

Auf diese Weise lassen sich aus dem ersten rohrförmigen Behälter, in welchem eine Gärung begonnen wird, im wesentlichen Wasserdampf, Kohlendioxyd und bereits Spuren von Alkohol abziehen. Bei Absenkung des Druckes auf das Niveau des zweiten rohrförmigen Behälters lassen sich bereits mit Alkohol angereicherte Dämpfe abziehen, wohingegen aus dem dritten Gärbehälter bis zum Erreichen eines zulässigen Restalkoholgehaltes weitgehend reiner Äthanol abgezogen werden kann. Die mehrstufige Verfahrensführung hat hiebei den Vorteil, daß die erhaltenen Produkte in höherer Reinheit anfallen.

Aus dem unter geringstem Druck stehenden Behälter kann nach Beendigung der Gärung Hefe abgezogen werden, wobei bevorzugt die aus dem unter geringstem Druck stehenden Behälter abgezogene Hefe zumindest teilweise gegebenenfalls über eine Konditionierungsstufe der Misch- und Trennvorrichtung rückgeführt ist.

Die Erfindung wird nachfolgend an Hand eines in der Zeichnung dargestellten Ausführungsbeispieles näher erläutert.

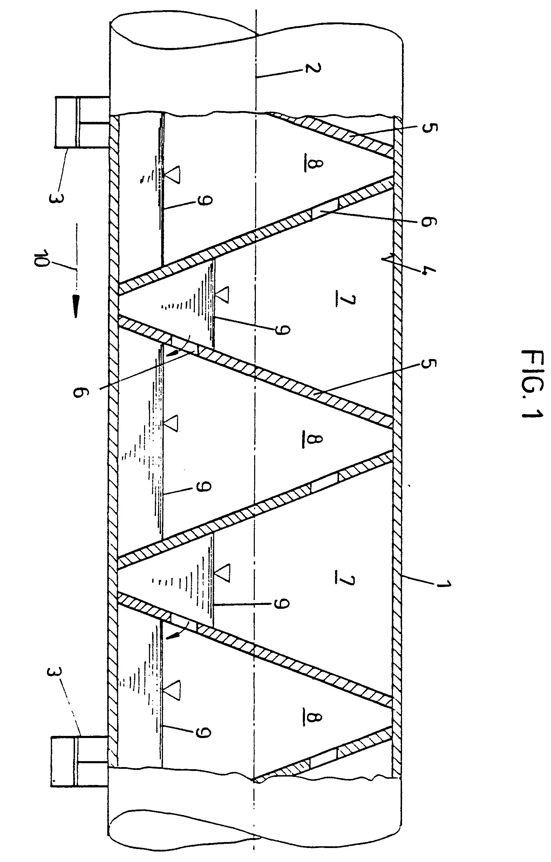

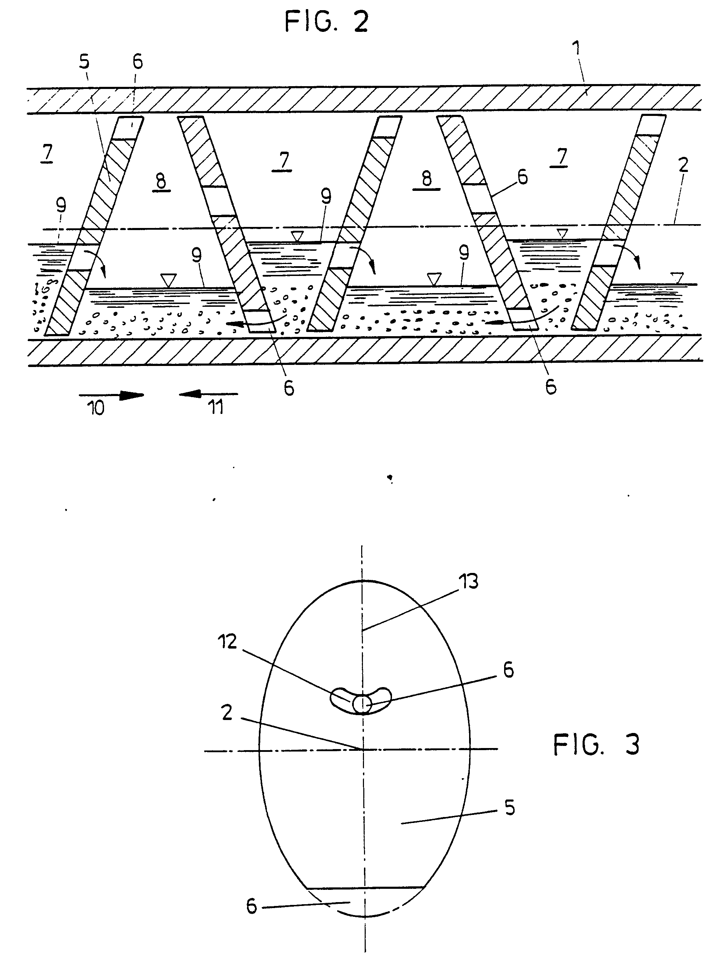

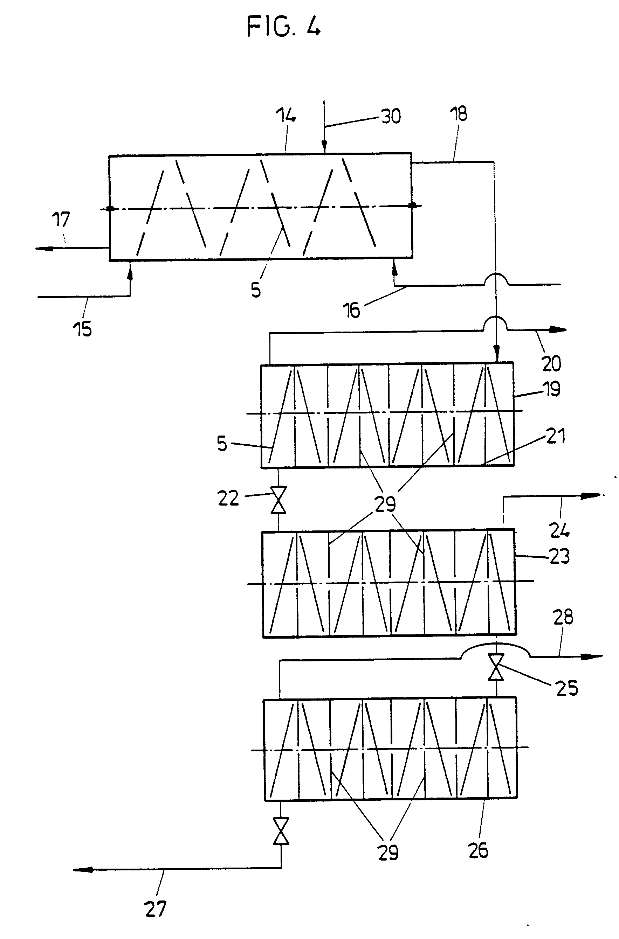

In dieser zeigen Fig. 1 einen Axialschnitt durch eine erste Ausbildung eines Gärbehälters, Fig. 2 eine Ausbildung einer Misch- und Trennvorrichtung gleichfalls im Axialschnitt, Fig. 3 eine Draufsicht auf eine Scheibe für die Vorrichtung nach Fig. 2 und Fig. 4 eine schematische Vorstellung der Einrichtung zur Herstellung von Alkohol aus gärfähigen Substraten, welche aus Einrichtungen entsprechend Fig. 1 und 2 zusammengesetzt ist.

In Fig. 1 ist ein Gärbehälter mit einem Rohr 1 dargestellt, welches um seine Achse 2 rotierbar in Lagern 3 gelagert ist. Der Innenmantel 4 des Rohres ist mit Scheiben 5 verbunden, welche im dargestellten Axialschnitt abwechselnd entgegengesetzt zueinander geneigt angeordnet sind. Jede dieser Scheiben 5 trägt nahe ihrem Umfang eine Durchbrechung 6, wobei die Durchbrechungen 6 in Richtung der Achse 2 gesehen jeweils um einen Winkel von 180° gegeneinander versetzt sind. Zwischen den benachbarten Scheiben 5 werden Kammern 7 und 8 ausgebildet, wobei jeweils in den Kammern 7 auf Grund der Stellung benachbarter Scheiben 5 der Flüssigkeitsspiegel 9 ansteigt, wohingegen er in den Kammern 8 auf Grund der verbreiterten Basis absinkt. Für den Transport zwischen benachbarten Kammern 7 und 8 bei Eintauchen der Durchbrechungen 6 in den Flüssigkeitsspiegel kann somit die hydrostatische Druckdifferenz ausgenützt werden und es erfolgt eine gerichtete Strömung in Richtung des Pfeiles 10.

Bei der Ausbildung einer Misch- und Trennvorrichtung nach Fig. 2 weist jede Scheibe 5 relativ zur gemeinsamen Drehachse 2 jeweils zwei Durchbrechungen 6 an bezüglich der Achse 2 gegenüberliegenden Seiten der Scheibe 5 auf. Die beiden Durchbrechungen 6 einer Scheibe sind somit bezüglich ihrer Wirksamkeit um 180° einer Umdrehung um die Achse 2 entgegengesetzt angeordnet und das Eintauchen der achsnahen Durchbrechungen 6 führt zu einem Überströmen des Mediums zwischen einer Kammer 7 und einer Kammer 8 im Sinne des Pfeiles 10, wohingegen das Eintauchen der achsfernen Durchbrechungen 6 eine entgegengesetzte Strömungsrichtung in Richtung des Pfeiles 11 zur Folge hat. Die Scheiben können wiederum mit dem Rohr 1 fest verbunden sein, sie können jedoch auch auf einer gemeinsamen Drehachse festgelegt sein, wobei dann an der Peripherie ein die Drehbarkeit sicherstellender Mindestabstand verbleibt, was zu einem gewissen Maß an alternierender Strömungsrichtung zwischen benachbarten Kammern 7 und 8 Anlaß gibt. Sofern der Drosselquerschnitt hinreichend klein ist, kommt es aber dennoch zu einer nennenswerten Anhebung des Flüssigkeitsspiegels und damit zu einem gerichteten Transport zweier Phasen in entgegengesetzte Richtungen. Die spezifisch leichtere Phase, wie beispielsweise flotierender Schlamm oder Schaum, wird auf Grund der achsnahen Durchbrechungen 6 in Richtung des Pfeiles 10 transportiert, wohingegen die spezifisch schwerere Phase nahe dem Mantel des Rohres im Sinne des Pfeiles 11 in die entgegengesetzte Richtung transportiert wird. Die beiden Stirnenden des Rohres 1 können hiebei dicht abgeschlossen sein und nicht dargestellte Zulauf- und Ablauföffnungen aufweisen. Der Ablauf für die spezifisch schwerere Phase wird hiebei in der Darstellung nach Fig. 2 am linken Ende des Rohres und der Ablauf für die spezifisch leichtere Phase, insbesondere Schaum oder flotierenden Schlamm, an der rechten Seite der Darstellung nach Fig. 2 vorgesehen.

Die einzelnen Scheiben, wie sie bei der Ausbildung nach Fig. 2 zum Einsatz gelangen, weisen die in Fig. 3 dargestellte Form auf. Es handelt sich hiebei im wesentlichen um elliptische Scheiben, wobei die achsferne Durchbrechung 6 als abgetrenntes Segment und die achsnahe Durchbrechung 6 als Bohrung ausgebildet ist. Die achsnahe Durchbrechung 6 kann ohne weiteres auch als Langloch 12 ausgeführt sein, wie dies in Fig. 3 angedeutet ist. In jedem Falle sind die einander bezüglich der Achse 2 gegenüberliegenden Durchbrechungen 6 der gleichen Scheibe mit Vorteil symmetrisch zur Hauptachse 13 der elliptischen Scheibe 5 zugeordnet.

Bei der schematischen Darstellung nach Fig. 4 ist eine Misch- und Trennvorrichtung entsprechend der Fig. 2 mit 14 bezeichnet. An diese Misch- und Trennvorrichtung mündet eine Zuleitung 15 für unbeladene Hefe und eine weitere Zuleitung 16 für zuckerreiche Lösung. Weiters ist eine Ableitung 17 für abgereicherte Zuckerlösung und eine Ableitung 18 für das Flotat, namentlich die beladene Hefe, vorgesehen. Auf Grund der im Zusammenhang mit Fig. 2 und 3 ausführlich dargelegten Strömungsverhältnisse läßt sich gleichzeitig mit einer intensiven Durchmischung der zuckerreichen Lösung mit der Hefe eine Trennung der flotierenden Hefe von der Zuckerlösung erzielen. Die beladene Hefe gelangt nun über die Leitung 18 in ein erstes Gärrohr 19, welches über eine Gasleitung 20 unter einen Druck von etwa 0,8 bar absolut gesetzt wird. Die innerhalb des Gärrohres 19 rotierbar gelagerten Scheiben 5 sind bei dieser schematischen Darstellung ohne Überströmöffnungen dargestellt. Vielmehr besteht zwischen dem Innenmantel 21 des Gärrohres 19 und dem Umfang der Scheiben 5 ein hinreichender Spalt, welcher eine gerichtete Strömung, beispielsweise durch Anwendung eines Saugdruckes an der der Aufgabe gegenüberliegenden Seite des Gärrohres 19, ermöglicht. Zu diesem Zweck ist das Gärrohr 19 über ein Absperrventil bzw. Drosselventil 22 mit einem zweiten nachfolgenden Gärrohr 23 verbunden, welches über eine Gasabzugsleitung 24 unter einen gegenüber dem Druck im Gärrohr 19 verringerten Druck gesetzt wird. Als Druck im zweiten Gärrohr 23 wird beispielsweise ein Druck von 0,3 bar absolut gewählt. Schließlich gelangt das an der gegenüberliegenden Seite abgezogene flüssige Medium über ein Absperr- und Drosselventil 25 in den nachfolgenden dritten Gärbehälter bzw. das Gärrohr 26. Nach Beendigung der Gärung kann die verwendete Hefe über eine Leitung 27 abgezogen werden und zumindest teilweise über die Leitung 15 der Misch- und Trennvorrichtung 14 rückgeführt werden.

Das dritte Gärrohr 26 wird beispielsweise unten einen Druck von etwa 0,15 bar absolut gesetzt, so daß aus diesem dritten Gärrohr 26 im wesentlichen reiner Alkohol abgezogen werden kann. Hiezu ist eine Gasabzugsleitung 28 vorgesehen.

Die einzelnen Gärbehälter können ohne weiteres wie in Fig. 4 dargestellt mit rotierenden Scheiben 5 ohne Durchbrechungen ausgebildet sein. In diesen Fällen sind starre Zwischenscheiben 29 angeordnet, wobei der achsnahe Bereich der Zwischenscheiben Durchströmöffnungen aufweist bzw. die Achse unter Freilassen eines Ringspaltes umschließt.

Mit 30 ist schematisch eine Zuleitung für Flockungshilfsmittel angedeutet.

## Patentansprüche

1. Einrichtung zur kontinuierlichen Gewinnung von Äthanol aus vergärbaren Zuckerlösungen mit einer Einrichtung zum Mischen von Zuckerlösung mit Hefe und zum Abtrennen von mit Zuckerlösung beladener Hefe und einem unter Unterdruck setzbaren Gärbehälter, dadurch gekennzeichnet, daß der Gärbehälter aus mehreren über Druckschleusen, insbesondere Ventile (22, 25) oder Pumpen, verbindbaren rohrförmigen Behältern (1, 19, 23, 26) gebildet ist, daß die einzelnen rohrförmigen Behälter (1) um die Achse (2) derselben drehbare Scheiben (5) aufweisen, welche zumindest teilweise in das Gärsubstrat eintauchen und mit der Achse (2) Winkel von größer oder kleiner 90° einschließen, und daß die rohrförmigen Behälter (1) gesonderte Gasableitungen (24, 28) aufweisen.

2. Einrichtung nach Anspruch 1, dadurch gekennzeichnet, daß den Behältern eine gleichfalls einen rohrförmigen Behälter aufweisende Misch- und Trennvorrichtung (14) vorgeschaltet ist, in welchem um die Behälterachse, gemeinsam mit dem Behälter oder gesondert, drehbare Scheiben (5) angeordnet sind, welche im wesentlichen dichtend an den Innenumfang des rohrförmigen Behälters anschließen, daß die Scheiben (5) in wenigstens einem Axialschnitt abwechselnd entgegengesetzt zur Achse (2) geneigt angeordnet sind und jeweils eine dem Innenumfang des Behälters nahe DurchstrÖmöffnung (6) und wenigstens eine der Achse (2) gegenüberliegende achsnahe Durchströmöffnung (6) aufweisen, wobei in Achsrichtung gesehen achsnahe bzw. dem Innenumfang nahe Durchströmöffnungen (6) an benachbarten Scheiben (5) um 180° in Drehrichtung versetzt angeordnet sind, und daß der Behälter an einem Ende eine Aufgabeöffnung (15) für Hefe und gegebenenfalls für Zuckerlösung und eine Austragsöffnung (17) für abgereicherte Zuckerlösung oder beladenen Hefeschlamm und am anderen Ende gegebenenfalls eine Aufgabeöffnung (16) für Zuckerlösung und eine Austragsöffnung (18) für beladenen Hefeschlamm oder für abgereicherte Zuckerlösung aufweist.

3. Einrichtung nach Anspruch 1 und 2, dadurch gekennzeichnet, daß die rohrförmigen Behälter des Gärbehälters die Eintrags- und Austragsöffnungen für beladene Hefe nahe den gegenüberliegenden Stirnseiten der Behälter aufweisen und daß die Scheiben (5) Überströmöffnungen (6) für den Transport des Gärsubstrates von der Eintragsöffnung zur Austragsöffnung aufweisen.

4. Einrichtung nach Anspruch 1, 2 oder 3, dadurch gekennzeichnet, daß der Druck in aufeinanderfolgenden rohrförmigen Behältern (19, 23, 26) stufenweise absenkbar ist.

5. Einrichtung nach einem der Ansprüche 1 bis 3, dadurch gekennzeichnet, daß die aus dem unter geringstem Druck stehenden Behälter (26) abgezogene Hefe zumindest teilweise der Misch- und Trennvorrichtung (14) rückführbar ist.

## Claims

1. An apparatus for the continuous extraction of ethanol from fermentable sugar solutions, having a device for mixing sugar solution with yeast and for separating yeast laden with sugar solution, and having a fermentation vessel which can be below atmospheric pressure, characterised in that the fermentation vessel is formed from a plurality of tubular vessels (1, 19, 23, 26) which can be connected via pressure gates, in particular valves (22, 25) or pumps, in that the individual tubular vessels (1) have discs (5) which can rotate about the axis (2) thereof and which are at least partly immersed in the fermentation substrate and form with the axis (2) an angle greater or smaller than 90°, and in that the tubular vessels (1) have separate gas outlets (24, 28).

2. An apparatus according to Claim 1, characterised in that upstream of the vessels there is disposed a mixing and separating device (14) which also has a tubular vessel in which are provided discs (5) rotatable about the vessel axis in common with or separately from the vessel, said discs adjoining the inner periphery of the tubular vessel in a substantially fluid-tight manner, in that in at least one axial section the discs (5) are arranged inclined alternately counter to the axis (2) and each have a throughflow opening (6) near to the inner periphery of the vessel and on the opposite side of the axis (2) at least one throughflow opening (6) near to the axis, wherein, viewed in axial direction, throughflow openings (6) respectively near to the axis or near to the inner periphery are provided in adjacent discs (5) offset by 180° in the direction of rotation, and in that at one end the vessel has a feed opening (15) for yeast and, optionally, for sugar solution and a discharge opening (17) for depleted sugar solution or laden yeast sludge, and at the other end it has, optionally, a feed opening (16) for sugar solution and a discharge opening (18) for laden yeast sludge or for depleted sugar solution.

3. An apparatus according to Claims 1 and 2, characterised in that the tubular vessels of the fermentation vessel have the feed and discharge openings for laden yeast near to the opposite end faces of the vessels, and in that the discs (5) have overflow openings (6) for the transfer of the fermentation substrate from the feed opening to the discharge opening.

4. An apparatus according to Claim 1, 2 or 3, characterised in that the pressure in successive tubular vessels (19, 23, 26) can be lowered in stages.

5. An apparatus according to any one of Claims 1 to 3, characterised in that the yeast drawn off from the vessel (26) in which the lowest pressure prevails can be returned at least partly to the mixing and separating device (14).

## Revendications

1. Dispositif de préparation en continu d'éthanol à partir de solutions sucrées fermentescibles, comportant un dispositif pour mélanger la solution sucrée de la levure, et pour séparer la levure chargée de solution sucrée, ainsi qu'un récipient de fermentation pouvant être mis sous dépression, caractérisé en ce que le récipient de fermentation est formé par plusieurs récipients tubulaires (1, 19, 23, 26) reliés par des sas de décompression, en particulier des vannes (22, 25) ou des pompes, en ce que les différents récipients tubulaires (1) présentent des disques (5) tournant autour de leur axe (2), qui pénètrent au moins partiellement dans le substrat fermentescible, et forment avec l'axe (2) des angles plus ou moins égaux à 90°, et en ce que les récipients tubulaires (1) comportent des départs de gaz (24, 28) séparés.

2. Dispositif selon la revendication 1, caractérisé en ce qu'en amont des récipients est monté un dispositif de mélange et de séparation (14), présentant aussi un récipient tubulaire, dans lequel sont disposés des disques (5) tournant autour de l'axe du récipient avec le récipient ou séparément, lesquels se raccordent de manière à peu près étanche sur le pourtour intérieur du récipient tubulaire, en ce que les disques (5) sont inclinés par rapport à l'axe (2) alternativement dans un sens et dans l'autre dans au moins une coupe axiale, et présentent chacun une ouverture de passage (6) à proximité du pourtour intérieur du récipient, et au moins une ouverture de passage (6) proche de l'axe, opposée à l'axe (2), les ouvertures de passage (6), proches de l'axe ou proches du pourtour intérieur, vues axialement, étant décalées de 180° en rotation, sur les disques (5) voisins, et en ce que le récipient comporte à une extrémité, une ouverture de chargement (15) pour la levure et le cas échéant pour la solution sucrée, ainsi qu'une ouverture d'évacuation (17) pour la solution sucrée appauvrie, ou la boue de levure chargée, et à l'autre extrémité le cas échéant une ouverture de chargement (16) pour la solution sucrée et une ouverture d'évacuation (18) pour la boue de levure chargée ou pour la solution sucrée appauvrie.

3. Dispositif selon les revendications 1 et 2, caractérisé en ce que les récipients tubulaires du récipient de fermentation, présentent les ouvertures de chargement et d'évacuation pour la levure chargée à proximité des faces frontales opposées des récipients, et en ce que les disques (5) présentent des ouvertures de trop-plein (6) pour le transport du substrat de fermentation depuis l'ouverture de chargement jusqu'à l'ouverture d'évacuation.

4. Dispositif selon les revendications 1, 2 ou 3, caractérisé en ce que la pression dans les récipients (19, 23, 26) tubulaires successifs s'abaisse progressivement.

5. Dispositif selon l'une des revendications 1 à 3, caractérisé en ce que la levure extraite du récipient (26) sous pression minimale, peut être renvoyée au moins partiellement au dispositif de mélange et de séparation (14).

FIG. 1

# FIG. 2

# FIG. 3

# FIG. 4